**Europäisches Patentamt**

**European Patent Office**

**Office européen des brevets**

(11) Veröffentlichungsnummer: **0 359 219**
**A1**

## (12) EUROPÄISCHE PATENTANMELDUNG

(21) Anmeldenummer: **89116926.0**

(22) Anmeldetag: **13.09.89**

(51) Int. Cl.⁵: **A61B 5/026**

(30) Priorität: **14.09.88 DE 3831216**

(43) Veröffentlichungstag der Anmeldung:
**21.03.90 Patentblatt 90/12**

(84) Benannte Vertragsstaaten:
**AT BE CH DE ES FR GB GR IT LI LU NL SE**

(71) Anmelder: **S & T Marketing AG**
**Zollstrasse 91**
**CH-8212 Neuhausen(CH)**

(72) Erfinder: **Acland, Robert D., Dr.**
**131 North Bayly Avenue**
**Louisville Kentucky 40206(US)**

(74) Vertreter: **Hiebsch, Gerhard F., Dipl.-Ing. et al**
**Hiebsch & Peege Patentanwälte Postfach**
**464 Erzbergerstrasse 5a**
**D-7700 Singen 1(DE)**

(54) **Verfahren und Vorrichtung zum Testen eines Blutgefaesses od. dgl.**

(57) Zur Untersuchung eines Blutgefäßes (G), das in der Rinne (20) eines Führungsblockes (12) positioniert werden kann, dient ein Lichtband (26), das durch die Enden (22) mehrerer, reihenförmig in der Rinnenwandung angeordneter Lichtleitfasern (24) erzeugt wird.

Fig. 2

EP 0 359 219 A1

# VERFAHREN UND VORRICHTUNG ZUM TESTEN EINES BLUTGEFÄSSES OD.DGL.

Die Erfindung betrifft ein Verfahren zum Testen eines Blutgefäßes od.dgl. mittels dieses beleuchtenden Lichtes. Zudem erfaßt die Erfindung eine Vorrichtung zum Testen eines Blutgefäßes, die insbesondere für das genannte Verfahren geeignet ist.

Das Beobachten von Blutgefäßen ist vor allem nach Operationen, Verletzungen bzw. beim Zusammenfügen von Gefäßenden erforderlich, um sicherzustellen, daß keine Blutgerinsel oder Thromben vorhanden sind, welche teilsweise oder gar ganz den Blutstrom unterbrechen könnten. Die Beobachtung muß während des chirurgischen Eingriffes und vor allem in den ersten 30 Minuten nach dem erneuten Fließen des Blutes erfolgen, da dies die Zeitspanne des größten Verstopfungsrisikos ist.

Bisher übliche Methoden, den Blutdurchfluß in beschädigt gewesenen Blutgefäßen zu testen, sind zum einen die Beobachtung des Pulsierens des Blutgefäßes sowie zum anderen einige Manipulationen, den Strom des Blutes erkennbar zu machen. Die bekannten Verfahren befriedigen nicht, da sie nur bei vollständigem Aussetzen des Blutstromes anzeigen, sie sind nicht ausreichend, um partielle Unterbrechungen erkennen zu lassen. Zudem sind Eingriffe mit Instrumenten stets gefährlich, darüberhinaus können die genauen Positionen der Blutgerinsel nicht fixiert werden.

Bei einer bisher versuchten Methode soll das Blutgefäß von der Gefäßinnenseite her beleuchtet werden. Dies hat sich als unpraktikabel erwiesen.

Angesichts dieser Gegebenheiten hat sich der Erfinder das Ziel gesetzt, ein betriebssicheres und einfach durchzuführendes Verfahren zu schaffen, mit dem einwandfreie Testergebnisse zu erzielen sind. Außerdem soll eine dafür geeignete Vorrichtung angeboten werden.

Zur Lösung dieser Aufgabe führt, daß mittels der Enden optisch leitender Fasern aus mehreren Lichtstrahlen ein Lichtband erzeugt, in einer Betrachtungsebene gegenüber einem optischen Aufnahmeorgan angeordnet und das Blutgefäß od.dgl. zwischen dem Aufnahmeorgan sowie dem -- linearen Lichtband hindurchgeführt bzw. positioniert wird. Das menschliche Auge als primäres Aufnahmeorgan steht exakt gegenüber dem Lichtband und kann so jede Veränderung des von außen her durchschienenen Blutgefäßes wahrnehmen, insbesondere den ganauen Sitz des Blutpfropfens und dessen von außen beleuchteten Umriß. Für dünne Blutgefäße kann das menschliche Auge ohne weiteres durch ein medizinisches Stereomikroskop unterstützt werden.

Bei einer für dieses Verfahren -- aber auch in anderer Weise -- einsetzbaren vorrichtung nach der Erfindung mündet das Ende einer optisch leitenden Faser -- bevorzugt mehrerer optischer Fasern -- in einer Aufnahmeeinrichtung für das Blutgefäß.

Die Faseroptik ist allgemein für Instrumente in der Medizin oder in der Nachrichtenverarbeitung bekannt, aber auch als eine besondere Art von Schmuck auf Stehlampen od.dgl. - derartige Lampen sind mit einem Büschel von Glasfasern versehen. Tritt ein Lichtstrahl in diese Fasern beispielsweise aus hochbrechendem Glas ein, wird er durch wiederholte Totalreflexion an einer niedrigbrechenden Glasmantelschicht zum anderen Faserende geleitet, so daß sich im Falle der geschilderten Lampe eine Vielzahl von Leuchtpunkten ergibt. In der Technik besonders bevorzugte Lichtleitfasern sind aus PMMA mit einem Mantel aus einem Polymeren niedrigen Brechungsindexes und mit Polyäthylenbeschichtung hergestellt.

Erfindungsgemäß dient als Aufnahmeeinrichtung eine in die Oberfläche eines Führungsblocks eingebrachte Rinne, in deren Wandung die optisch leitenden Fasern enden. Bevorzugt verläuft die Rinne in der Blockoberfläche quer zur Längsachse des Führungsblockes. In die Rinne ist ein parallel zur Rinnenachse verlaufendes Band aus mehreren Enden von optisch leitenden Fasern integriert und liegt in vorteilhafter Ausführung im Rinnentiefsten.

Als günstig hat eine Konfiguration erwiesen, bei der die Faserenden in mehreren linearen Reihen angeordnet sind, wobei die Faserenden der einen Reihe sich in die Einschnürungen der benachbarten Reihe einschmiegen können.

Um den optischen Kontrast zum Lichtband zu verbessern, soll der Führungsblock eine schwarze Oberfläche aufweisen, gegebenenfalls sogar insgesamt aus dunkelfarbigem, insbesondere aus schwarzem Werkstoff gefertigt sein.

Der Führungsblock besteht nach einem weiteren Merkmal der Erfindung aus kaltverformtem Epoxyharz, da die eingebetteten optischen Fasern selbst sehr temperaturempfindlich sind und bei der Herstellung der Vorrichtung nicht zerstört werden dürfen.

Der Führungsblock verjüngt sich zu seinem freien Ende hin bevorzugt zu einer parallel zur Aufnahmeeinrichtung oder Rinne verlaufenden Kammlinie, um leicht unter das Blutgefäß geschoben werden zu können.

Nach weiteren erfindungsgemäßen Merkmalen bestehen die optisch leitenden Fasern aus Polystyrol und verlaufen in einer ummantelten Lichtleitung zum Führungsblock hin, in dem das Ende der Lichtleitung festgelegt ist. Innerhalb des Führungsblockes sind die optisch leitenden Fasern aus der Achse der Lichtleitung zur Rinne hin gekrümmt, dies mit einem Krümmungsradius von etwa 0,6 mm; dieses Maß gewährleistet einen optimalen Lichttransport in die Rinne.

Weitere Vorteile, Merkmale und einzelheiten der Erfindung ergeben sich aus der nachfolgenden Beschreibung eines bevorzugten Ausführungsbeispieles sowie anhand der Zeichnung; diese zeigt in

Fig. 1: eine Schrägsicht auf eine Vorrichtung zum Betrachten von Blutgefäßen bzw. Abschnitten davon;

Fig. 2: die gegenüber Fig. 1 vergrößerte Seitenansicht eines teilweise geschnitten dargestellten Teiles der Fig. 1;

Fig. 3: die teilweise geschnittene Draufsicht auf den Gegenstand der Fig. 2;

Fig. 4: einen vergrößerten Ausschnitt aus Fig. 3.

Eine Vorrichtung 10 zum Prüfen von Blutgefäßen G insbesondere nach operativen Eingriffen od.dgl. besteht aus einem Führungsblock 12 -- einer beispielsweisen Länge n von 11 mm, einer Breite b von 3 mm sowie einer Höhe h von 2 mm -- einerseits und einer flexiblen Lichtleitung 14 anderseits, die an eine bei 16 angedeutete Lichtquelle angeschlossen ist.

Nahe des sich zu einer Kammlinie 17 verjüngenden freien Blockendes 18 ist in die Blockoberfläche 19 eine Querrinne 20 halbkreisförmigen Querschnittes mit dem Durchmesser d von hier 1,2 mm etwa parallel zu jener Kammlinie 17 eingebracht.

Dieses Maß ist als Durchschnittsvorgabe anzusehen; der Rinnendurchmesser d wird entsprechend dem Querschnitt der zu behandelnden Gefäßart ausgewählt.

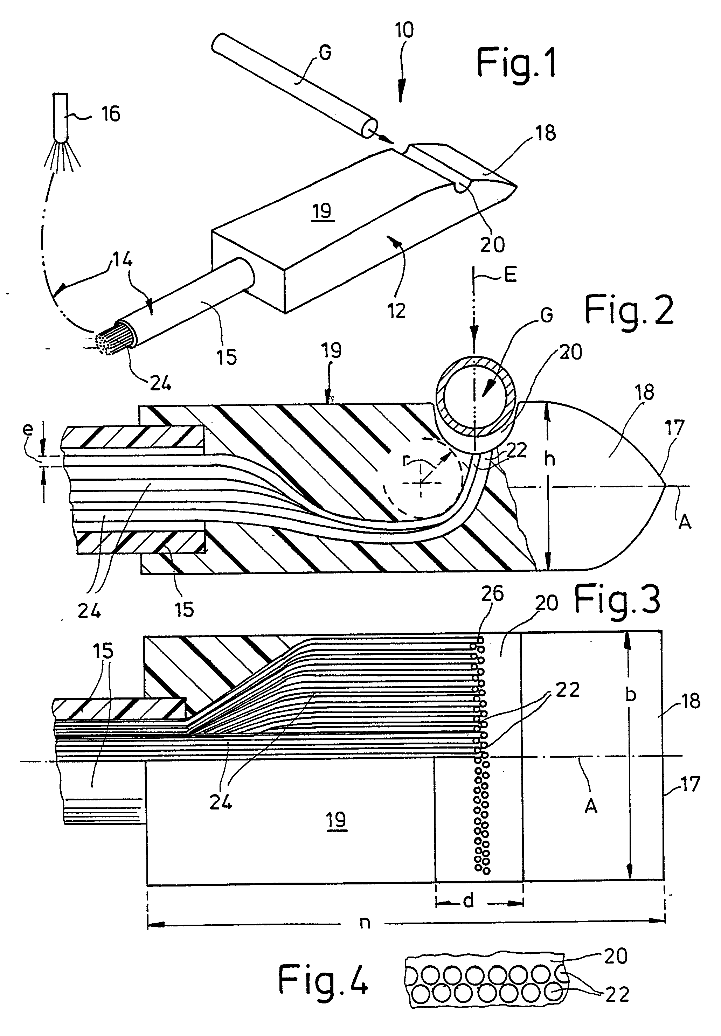

Im Tiefsten der Querrinne 20 münden hier zwei Reihen von Enden 22 optisch leitender Fasern 24 als Lichtband 26. Dabei sind die Faserenden 22 der einen Reihe gegenüber jenen der anderen Reihe so versetzt, daß sie sich in die durch jeweils zwei kreisförmige Fasermündungen begrenzte Einschnürungen einschmiegen (Fig. 4).

Die Fasern 24 sind gemäß Fig. 2 im Führungsblock 12 in einem Krümmungsradius r (beispielsweise 0,6 mm) in die Blockachse A umgelenkt und verlaufen dann in einem Mantel 15 jener Lichtleitung 14.

Die genannte Radiusgröße ist für den ungehinderten Transport des Lichtes von jener Lichtquelle 16 durch die optisch leitenden Fasern 24 zur Querrinne 20 bzw. deren Lichtband 26 erforderlich; ein zu testendes Blutgefäß G wird vom Führungsblock 12 unterfahren sowie in die Querrinne 20 eingelegt und dann in einer optisch günstigen Betrachtungsebene E -- senkrecht zum Lichtband 26 -- beobachtet.

Der beschriebene Auflageblock 12 ist aus Gründen eines besseren optischen Kontrastes schwarz gehalten und -- zur Meidung von Temperatureinflüssen auf die eingegossenen optischen Fasern 24 -- aus Epoxyharz kalt geformt. Die optischen Fasern 24 eines bevorzugten Durchmessers e von 0,007 mm bestehen aus Polystyreen, der Mantel 15 ihrer Lichtleitung 14 aus linearen aliphatischen Polyamiden beispielsweise der Marke Nylon.

Es wird ohne weiteres erkennbar, daß das helle Lichtband 26 aus den Faserenden 22 in dem an sich schwarzen Auflageblock 12 die Lichtkonzentration während des Betrachtungsvorganges begünstigt. Das in das Tiefste der Querrinne 20 geleitete Licht reicht völlig aus, das Blutgefäß G durchscheinend werden zu lassen, so daß eine genaue Betrachtung der Verhältnisse am Blutgefäß G möglich wird.

## Ansprüche

1. Verfahren zum Testen eines Blutgefäßes od.dgl. mittels dieses beleuchtenden Lichtes, dadurch gekennzeichnet, daß mittels der Enden optisch leitender Fasern aus mehreren Lichtstrahlen ein Lichtband erzeugt, in einer Betrachtungsebene gegenüber einem optischen Aufnahmeorgan angeordnet und das Blutgefäß od.dgl. zwischen Aufnahmeorgan sowie Lichtband hindurchgeführt bzw. positioniert wird.

2. Vorrichtung zum Testen eines Blutgefäßes mit Licht, insbesondere nach dem Verfahren des Anspruches 1, dadurch gekennzeichnet, daß zumindest das Ende (22) einer optisch leitenden Faser (24) in einer Aufnahmeeinriohtung (20) für das Blutgefäß (G) mündet.

3. Vorrichtung nach Anspruch 2, gekennzeichnet durch einen Führungsblock (12) mit in dessen Oberfläche (19) eingebrachter Rinne (20) als Aufnahmeeinrichtung, in deren Wandung die optisch leitende/n Faser/n (24) endet/enden, wobei gegebenenfalls die Rinne (20) in der Blockoberfläche (19) quer zur Längsachse (A) des Führungsblockes (12) verläuft.

4. Vorrichtung nach Anspruch 1 oder 2, dadurch gekennzeichnet, daß ein parallel zur Rinnenachse verlaufendes Band (26) aus mehreren Enden (22) von optisch leitenden Fasern (24) vorgesehen ist, welches gegebenenfalls aus Faserenden (22) im Rinnentiefsten verläuft.

5. Vorrichtung nach einem der Ansprüche 2 bis 4, dadurch gekennzeichnet, daß das Lichtband (26) aus wenigstens zwei nebeneinander verlaufenden Reihen von Faserenden (22) gebildet ist, wobei die Faserenden (22) einer Reihe denen der anderen Reihe versetzt anliegen.

6. Vorrichtung nach wenigstens einem der Ansprüche 2 bis 5, dadurch gekennzeichnet, daß der Führungsblock (12) eine schwarze Oberfläche (19) aufweist und oder der Rinnendurchmesser (d) etwa 1,2 mm beträgt.

7. Vorrichtung nach wenigstens einem der Ansprüche 1 bis 6, dadurch gekennzeichnet, daß der Führungsblock (12) aus dunkelfarbigem, insbesondere aus schwarzem Werkstoff gefertigt ist und oder aus kaltverformten Epoxyharz besteht.

8. Vorrichtung nach wenigstens einem der Ansprüche 2 bis 7, dadurch gekennzeichnet, daß der Führungsblock (12) sich zu seinem freien Ende hin verjüngt und oder, daß der Führungsblock an seinem verjüngten Ende eine parallel zur Aufnahmeeinrichtung oder Rinne (20) verlaufende Kammlinie (18) bildet.

9. Vorrichtung nach wenigstens einem der Ansprüche 2 bis 8, dadurch gekennzeichnet, daß die optisch leitenden Fasern (24) aus Polystyrol bestehen und in einer ummantelten Lichtleitung (14) zum Führungsblock (12) verlaufen.

10. Vorrichtung nach wenigstens einem der Ansprüche 2 bis 9, dadurch gekennzeichnet, daß die optisch leitenden Fasern (24) aus der Achse der Lichtleitung (14) zur Rinne (20) od.dgl. hin gekrümmt sind und oder daß der Krümmungsradius (r) der optisch leitenden Fasern (24) im Führungsblock (12) etwa 0,4 bis 0,8 mm, vorzugsweise 0,6 mm beträgt.

Fig.1

Fig.2

Fig.3

Fig.4

Europäisches Patentamt

**EUROPÄISCHER RECHERCHENBERICHT**

Nummer der Anmeldung

EP 89 11 6926

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-B-2 517 129 (SIEMENS AG)<br>* Spalte 4, Zeilen 5-16; Figur 5 * | 1,10 | A 61 B 5/026 |
| A | EP-A-0 280 418 (WYATT TECHNOLOGY CORP.)<br>* Zusammenfassung; Spalte 6, Zeilen 41-47; Spalte 7, Zeilen 10-25; Spalte 8, Zeilen 26-32; Spalte 8, Zeile 47 - Spalte 9, Zeile 12; Figuren 1,3,5 * | 1,4 | |
| A | FR-A-2 571 604 (I.N.S.E.R.M.)<br>* Zusammenfassung; Seite 3, Zeilen 6-18; Seite 4, Zeilen 18-24; Seite 5, Zeilen 25-29; Figuren 1,2 * | 1 | |
| A | US-A-3 602 213 (W.L. HOWELL et al.)<br>* Zusammenfassung; Spalte 11, Zeile 10 - Spalte 12, Zeile 28; Figuren 1-8 * | 1,4,5,7 | |
| A | GB-A-2 068 537 (ENERGY CONVERSION DEVICES INC.)<br>* Zusammenfassung; Seite 2, Zeilen 87-119; Seite 5, Zeilen 74-116; Figuren 5-7 * | 1,4,5 | **RECHERCHIERTE SACHGEBIETE (Int. Cl.5)**<br><br>A 61 B |

Der vorliegende Recherchenbericht wurde für alle Patentansprüche erstellt

| Recherchenort | Abschlußdatum der Recherche | Prüfer |
|---|---|---|
| DEN HAAG | 05-12-1989 | RIEB K.D. |